# EUROPEAN PATENT APPLICATION

(11) **EP 2 052 745 A1**
(43) Date of publication of application: **29.04.2009**
(21) Application number: 09151507.2
(22) Date of filing: 22.02.2007
(51) Int. Cl.: A61L 9/03

(54) **Air treatment device with heated volatile dispenser**

(30) Priority: 22.02.2006 US 359090
(62) Divisional of application: 07751220.0
(71) Applicant: S.C.JOHNSON & SON, INC., Racine, WI 53403-2236 (US)
(72) Inventor: Davis, Brian T., Burlington, WI 53105 (US); Adair, Joel E., Racine, WI 53402 (US); Saleh, Saleh A., Vermon Hills, IL 60061 (US); Emmrich, Robert R., Racine, WI 53402 (US)
(74) Representative: Ruschke, Hans Edvard

(57) **Abstract**

Disclosed is a device suitable for dispensing air treatment chemicals by heating a substrate impregnated with the chemicals. The device has a heater in the form of a table where a face of the table radiates heat directly to the impregnated substrate. The substrate releases the air treatment chemical back towards the heater. Also disclosed are methods for assembling such devices.

## Description

### CROSS-REFERENCE TO RELATED APPLICATIONS

Not applicable.

### STATEMENT REGARDING FEDERALLY SPONSORED

### RESEARCH/DEVELOPMENT

Not applicable

### BACKGROUND OF THE INVENTION

The present invention relates to devices that dispense a volatile air treatment chemical by heating a substrate that is impregnated with, or coated with, the chemical. More particularly it relates to such devices that efficiently use heat and are easily assembled.

Substrates (particularly porous substrates) have previously been used as carriers for air treatment chemicals such as insect control agents (insecticides, insect repellents, insect growth regulators, attractants, synergists, etc.), fragrances and deodorizers. See e.g. U.S. patent 6,551,560. The disclosure of this patent, and of all other patents referred to herein are incorporated by reference as if fully set forth herein.

Upon heating of the substrate a volatile air treatment chemical is caused to be dispensed from the substrate. The heating source is typically an electrical heater, but may instead be a flame in some cases.

A variety of air treating functions can be achieved with such devices. For example a porous substrate impregnated with volatile insecticide can be used to inhibit mosquito biting in a confined bedroom. Alternatively, a deodorizing or other odor control material can be dispensed to overcome malodors, or to simply provide a desired fragrance.

One problem with such devices is that the substrate usually rests against a heater. The heater heats the substrate, causing the volatized air treatment chemical to be driven off the substrate in a direction away from (essentially perpendicular to) a heater surface. This can create inefficiencies. For example, the portion of the substrate adjacent the heater can act as an insulator for the portion of the substrate which is releasing the active, making control of the dispensing more difficult.

Another problem with such devices is that it may take too long after usage begins to adequately treat the air in a defined environment adjacent the device. For example, when someone is about to go to bed they may activate the device in a bedroom. It is undesirable for them to have to wait a long period before feeling secure about insect protection in the room. If the heater must heat the entire vertical expanse of the substrate prior to dispensing being most efficient from a surface directed away from the heater, that can delay the start-up coverage.

In other developments U.S. patent application publication 2002/0066798 disclosed the use of a type of snap fit connection in assembling a scent dispenser. However, that assembly was unduly complex.

Thus, to date prior art heating dispensers for dispensing air treatment chemicals were not optimal with regard to how heat was applied to the substrate or how the construction was assembled. Devices which addressed these concerns therefore continue to be desired.

### BRIEF SUMMARY OF THE INVENTION

In one aspect the invention provides an air treatment chemical dispensing device that can, upon mounting a substrate bearing a volatile air treatment chemical thereto, dispense the air treatment chemical from the substrate. The device has a housing and a heater positioned in the housing. The heater is in the form of a table having a facing wall, and is capable of radiating heat from at least the facing wall. There are means for mounting the substrate adjacent the facing wall so that when heat is radiated from the facing wall it will radiate at least in part towards the substrate to cause the substrate to release the air treatment chemical at least partially towards the facing wall.

The mounting means is preferably a mechanical one (e.g. resilient fingers). However, it could be any other suitable means for mounting such as adhesives, fasteners and other mechanical retainers.

In any event, there is also an air pathway structure in and through the housing permitting air to pass into the housing between the facing wall and the substrate (if such a substrate is mounted in the housing by the mounting means), and then out of the housing.

In a particularly preferred form the heater also has a side wall structure capable of radiating heat there from into a portion of an air pathway structure. This is used with an air pathway structure that has an inlet vent and an outlet vent, these vents being preferably located on opposed lateral sides of the device. In this manner air can be drawn in one vent, pass partially across the lateral sides of the heater, pass partially across the facing wall of the heater, and then out the outlet. The air near the substrate will mix with the released air treatment chemical. The treated air will then be released out the outlet.

This transverse air pathway is particularly desirable because the flow is at least in part between the heater and the substrate. Also, because the inlet vent can be at the bottom of the device (as the device is positioned when plugged into a vertical outlet), the heated air will naturally rise towards the outlet, further improving flow efficiency.

When the substrate is mounted as in this manner, it is most preferred that it have a projecting nose positioned in contact (or near contact) with the facing wall. The remainder of the substrate will not be in contact with the heater. Thus, heat can be directly transferred to the nose, while additional heated air passes around the nose between the substrate and the heater.

The volatile air treatment chemical can be selected from the group consisting of insect control agents, fragrances, and deodorizers. For example the volatile air treatment chemical can be an insect control agent which is selected from the group consisting of natural pyrethrins, pyrethrum extract, synthetic pyrethroids, other volatile insect control agents, and mixtures thereof.

In another form the invention provides an air treatment chemical dispensing device that can, upon mounting a substrate bearing a volatile air treatment chemical thereto, dispense the air treatment chemical from the substrate. The device has a housing, an electrical plug structure mounted in the housing with a portion extending rearwardly outward there from, a heater linked to the electrical plug structure in the housing, and a cover unit for covering the heater which is mountable to the housing. The cover unit is configured to be suitable to mount the substrate such that the substrate has a first wall of the substrate facing the heater while a second wall of the substrate, on a side of the substrate opposite to the first wall, is essentially covered by the cover unit.

Such a structure permits easy assembly which can be automated at low cost. For example, the housing, electrical plug structure, heater and cover unit can be assembled by relative axial movement there between, preferably with a snap fitting telescoping connection as part of the assembly.

In yet another form, the invention provides methods of assembling such devices. One can place the electrical plug structure in the housing with a portion extending rearwardly outward there from. One then places the heater in the housing and moves it axially until it telescopes with the electrical plug structure. One then mounts the cover unit to the housing while the cover unit has the substrate positioned thereon.

The substrate is preferably coated with or impregnated with a volatile air treatment chemical. In a most preferred version the substrate is configured such that it tends to wick air treatment chemical towards the heater. This could occur because the substrate comprises granular sand particles bound to a phenolic resin, where the particle size is smaller at the portion of the substrate adjacent the heater.

It should be appreciated that the devices of the present invention are inexpensive to produce, and use heat extremely efficiently. The foregoing and other advantages of the present invention will be apparent from the following description. In the description that follows reference is made to the accompanying drawings which form a part thereof, and in which there is shown by way of illustration, and not limitation, expected preferred embodiments of the invention. Such embodiments do not necessarily represent the full scope of the invention, and reference should therefore be made to the claims herein for interpreting the scope of the invention.

### BRIEF DESCRIPTION OF THE DRAWINGS

FIG. 1 is a frontal, right perspective view of an air treatment device of the present invention, plugged into a vertical wall;

FIG. 2 is a sectional view taken along line 2-2 of FIG. 1, albeit with an indicator unit 26 removed;

FIG. 3 is an exploded view of the FIG. 2 device;

FIGS. 4A-4C illustrate a portion of a preferred method of assembly of a housing, an electrical plug structure, and a heater of the present invention; and

FIGS. 4D and 4E illustrate further steps of assembly of a device of the present invention.

### DETAILED DESCRIPTION OF THE PREFERRED EMBODIMENTS

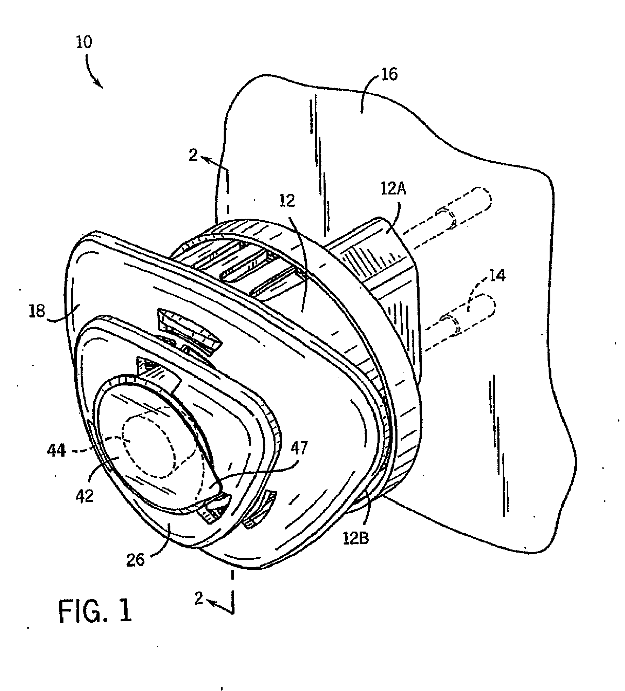

Referring first to FIG. 1, an air treatment device 10 has an outer housing 12 comprising a rear portion 12A and a frontal portion 12B. An electrical prong structure 14 is positioned in the housing 12, in the rear portion 12A, and has a rearward end extending rearwardly outwardly there from.

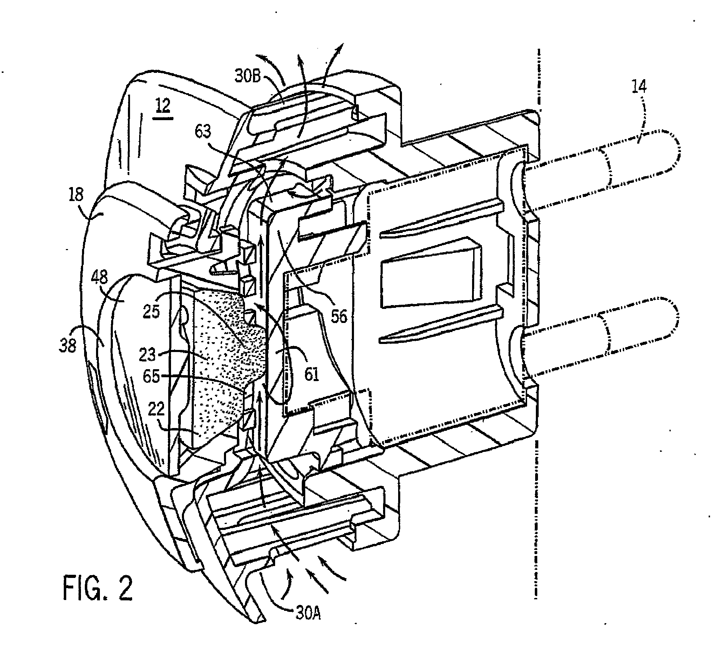

There is also a cover unit 18 mounted to the frontal housing portion 12B. The cover unit 18 mounts a substrate 22 (see especially FIG. 2) such that the substrate is essentially outwardly frontally covered, but is open towards the interior of the housing.

In a particularly preferred version there is a separately installable indicator unit 26 removably mountable to the cover unit 18 so as to project outwardly and forwardly. The indicator unit 26 is preferably removable from the cover unit 18, and houses a separate indicator chemical in a cup-shaped structure 44, which may indicate to a user the amount of air treatment chemical remaining in the substrate 22. The indicator unit 26 may have a removable lid 42 with an easy-grab tab 47. The rear of the indicator unit 26 may extend into a well 38 formed in the cover unit to facilitate some heat transfer through wall 48 to the indicator unit.

The device 10 is most preferably plugged into an electric socket on a vertical wall 16. The directional terms in this patent are used with that type of installation in mind. However, appropriate electric sockets on horizontal or other surfaces may also be used to provide power. Thus, the terms such as "front", "rear", "upper", "lower", and "side" should be interpreted in an analogous manner when the devices are used for that type of installation.

The electrical prong structure 14 shown in the figures are merely for purposes of example. Cylindrical prongs of this type are suitable for linking to electric power in some countries. However, in other countries blade prongs, or mixtures of blades, cylinders and other shaped prong elements will be used to supply the linkage to the available power (as is well recognized in the art).

The frontal housing 12 has a series of elongated vents 30 on its upper and lower sides. The vents 30A form an inlet part of an air pathway, by allowing air from the environment to enter. Air then passes as shown by the arrows in FIG. 2.

Note that the heater 56 is in the form of a table having a frontal facing wall 61 and a side wall structure 63. Heat can radiate towards the substrate 22, and also sideways around the table. Thus, air entering the vents 30A will heat up very quickly and efficiently.

Note also that the nose projection 25 on the substrate 22 can be in direct contact with the facing wall 61. This permits direct heat transfer. Nevertheless, additional heat can be applied along the sides of the nose as the somewhat heated air passes between the wall 65 of the substrate that faces the table and the facing wall 61.

This is particularly effective in causing a very fast burst of insecticide or other air treatment chemical when the device is first turned on. Hence, a room can be rendered adequately treated quite quickly. Also, where the substrate 22 is of the type that wicks the air treatment chemical towards the wall 65, the burst can be repeated after the device has been shut off for a day and then turned on again.

After the air treatment chemical has been released into the air adjacent facing wall 61, it will pass generally transversely along the facing wall 61 until it exits outlet vent 30B. This then treats the surrounding environmental air with the air treatment chemical.

While the preferred substrate shape is a substrate having a forward frustum shaped section 23 and a rearward projecting nose 25, other shapes can be used, with or without a projecting portion. The substrate 22 is preferably completely impregnated with a volatile air treatment chemical capable of being dispensed from the substrate 22 when the substrate 22 is heated. However, as an alternative to being completely impregnated with the air treatment chemical, the substrate 22 may instead be only partially impregnated or just coated with the chemical.

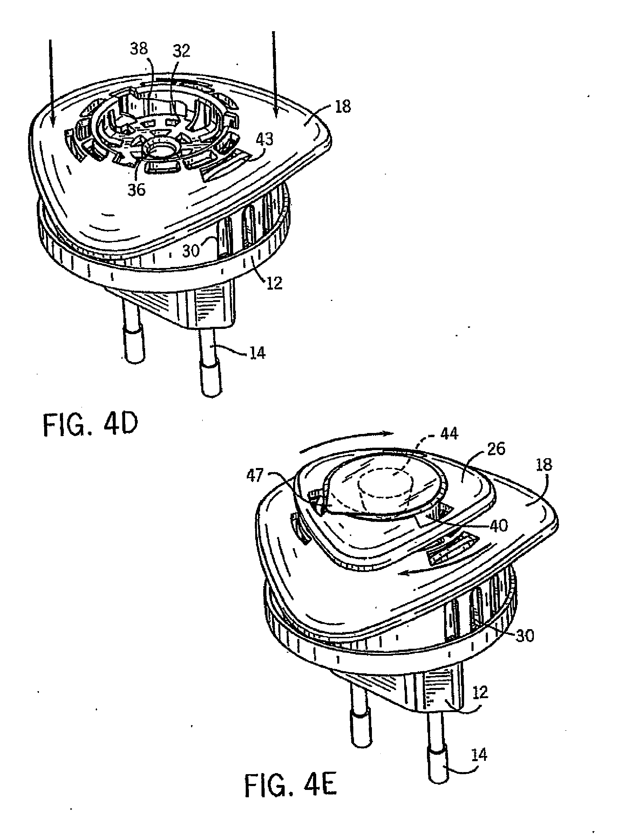

The housing 12 of the overall device 10 encloses the table-shaped heater with a sufficient insulation gap to the outer housing wall to prevent the side wall from heating too much. The heater is preferably activated by inserting the rearward end of the electrical prong structure 14 into an outlet. Heat from the heater 56 may also be permitted to pass against other surfaces of the cover unit 18 through a series of openings 32 and 36 (see FIG. 4D). Note that wall 48 effectively closes off air dispensing through the front of the device.

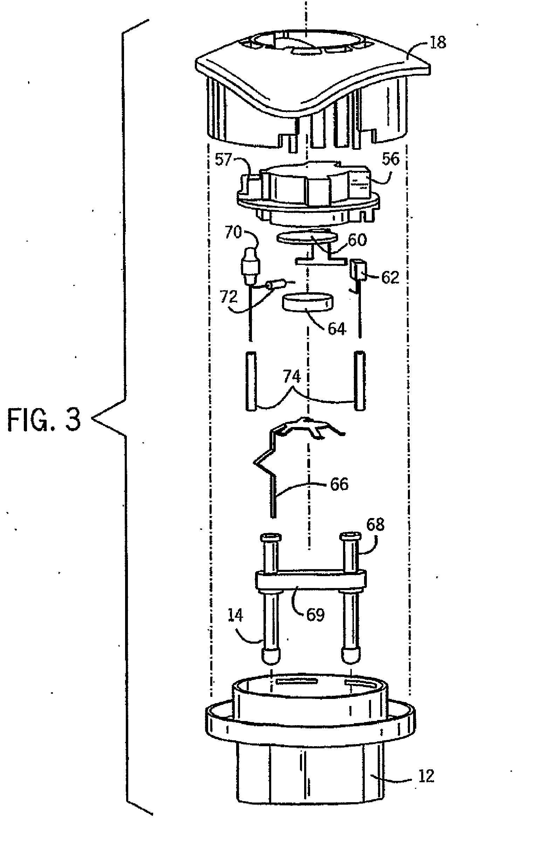

Referring next to FIG. 3, from top to bottom (forward to rear in the installed device), the device 10 has a removable cover unit 18 (shown here without the indicator unit 26) which, lockingly engages with the heater 56, in a subassembly, after positioning the substrate in the cover unit. This can be achieved with a snap fit connection, or by a bayonet connection, or by other means.

A heater enclosure wall 56A is linked to an aluminum contact 60, a thermal cutoff (TCO) 62, a thermistor 64, a neon in use light 70, and a resistor 72, all of which telescopingly, matingly engage with the underside of the heating enclosure wall 56A. The TCO 62 and light 70 each have an end which matingly engage with corresponding silicon sleeves 74, which in turn snap into corresponding holes in the housing 12. A star contact 66 is preferably inserted between the aluminum contact 60 and one of two plug decks 68 forming a pin bridge 69. In turn, the pin bridge 69 preferably snaps into the housing 12, thereby completing the device 10.

It should be appreciated that the main components of the assembly can be assembled without tools, and in most cases with simple axial relative movement. This makes the production of the device extremely inexpensive.

Once the device is plugged in, the electric current moves through the electrical prong structure 14 to drive the heater and the on light 70. Overheating is prevented by the TCO.

The heater enclosure wall 56A may be made of any material suitable for the environment (e.g. heat resistant metals, plastics and the like). While the heater can be of many forms, a resistance heater is preferred. However, in some forms a flame, catalytic burner, or other combustion source may heat the table.

Further, while a variety of resistance elements may be used, we prefer a low resistance thermistor 64, which has a positive temperature coefficient in which the zero-power resistance increases with an increase in temperature.

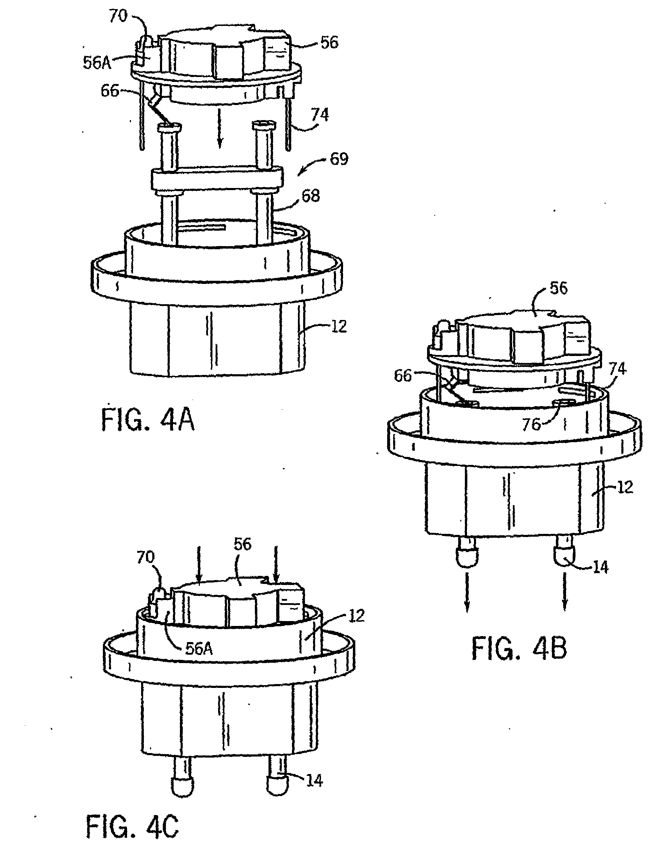

Referring next to FIGS. 4A-4E, the telescopic, snap-fit assembly method of the device 10 is depicted. In the most preferred form each piece of the device 10 preferably telescopically fits together, and in some cases the parts snap fit together to lock the assembly together. In use, the on light 70 telescopingly engages an opening 57 within the heater enclosure wall 56A configured to accommodate the on light 70. When the light 70 is properly positioned in the opening 57, the light 70 snaps into position, thus securing the light 70 to the heater enclosure wall 56A. When the light 70 is snapped in place, the star contact 66 is telescopingly inserted into a plug deck 68 of the pin bridge 69 and snapped in place. At the same time, the pin bridge 69 is inserted into the housing 12 and snapped in place.

The electrical prong structure 14 includes a bridge 69. The electrical prong structure is pulled through the rearward end of the housing 12 until the prongs snap in place (see FIG. 4B). This pulls the heater enclosure wall 56A over the contact 60, TCO 62, thermistor 64 and resistor 72. The heater enclosure wall 56A is preferably pushed down towards the housing 12, past retaining beads (not shown) until the heater enclosure wall 56A snaps into place in the housing 12 (see FIG. 4C).

Once the heater enclosure wall 56A is secured in the housing 12, the cover unit 18 (with the substrate 22 included) is preferably inserted into corresponding openings on the housing (not shown) by pressing firmly downward until the cover unit 18 snaps into place. Finally, an optional indicator unit 26 or other decorative plate (not shown) may be screwed into place on the front of the cover unit 18 by engaging the legs 40 of the indicator unit with corresponding openings 43 defined by the cover unit 18.

Similarly, legs (not shown) of the cover unit 18 permit a quick, snap-fit attachment to the housing 12 via corresponding grooves 50 in the housing 12. See e.g. FIG. 4E.

Examples of a suitable substrate 22 include but are not limited to porous sand with a binder such as novolac resin, urethane resins or highly cross linked thermoplastics such as cross linked polyethylene. Particularly preferred sand substrates can be made in a fashion analogous to the sand wicks described in U.S. patent application publication 2005/0284952. Alternative substrates include other particulates such as metal, cellulose, and ceramic particulates.

The air treatment chemical is preferably an insecticide, fragrance and/or disinfectant. In some cases more than one air treatment chemical may be used alone or in combination in the substrate 22.

When the air treatment chemical is an insecticide and/or insect repellent, organic phosphorous insecticides, lipidamide insecticides, natural repellents as citronella oil, natural pyrethrins and pyrethrum extract, and synthetic pyrethroids are preferred. Suitable synthetic pyrethroids are acrinathrin, allethrin as D-allethrin, PynaminR^{™}, benfluthrin, bifenthrin, bioallethrin as Pynamin ForteR^{™}, S-bioallethrin, esbiothrin, esbiol, bisoresxnethrin, cycloprothrin, cyfluthrin, beta-cyfluthrin, cyhalothrin, lambda-cyhalothrin, cypermethrin, alpha-cypermethrin, beta cypermethrin, cyphenothrin, deltamethrin, empenthrin, esfenvalerate, fenpropathrin, fenvalerate, flucythrinate, taufluvalinate, kadethrin, permethrin, phenothrin, prallethrin as EtocR^{™}, resmethrin, tefluthrin, tetramethrin, tralomethrin, metofluthrin, or transfluthrin. Other volatile insecticides, such as those described in U.S. patent 4,439,415, can also be employed.

In particularly preferred versions the volatile insecticide is selected from the group consisting of transfluthrin, metofluthrin, vapothrin, permethrin, prallethrin, tefluthrin and esbiothrin. Transfluthrin is the most preferred insecticide.

Possible solvents for carrying these air treatment chemicals include, but are not limited to, ISOPAR^{™}C, ISOPAR^{™}E, ISOPAR^{™}L, heptane, methanol, acetone, ethanol, isopropyl alcohol, dodecene and tetraydrafuran. ISOPAR^{™}C, ISOPAR^{™}E and ISOPAR^{™}L are hydrocarbon solvents of varying chain length and are available from Exxon Chemical Company.

Typically, volatile insect control agents will be carried in an organic solvent such as a hydrocarbon. One particularly desirable impregnation formulation for mosquito control is 50 wt. percent transfluthrin dissolved in ISOBAR C, hydrocarbon. Alternatively and often preferably, transfluthrin can first be warmed to liquefy it and then applied neat to a warmed substrate.

A wide variety of volatile fragrances may be used which may optionally also have insect control attributes. Alternatively, some fragrances may be selected that provide a deodorizing function (e.g. certain terpenes). For example, various natural and artificial perfumes may be used. Non-limiting examples of these perfumes include animal-based and plant-based natural perfumes, and artificial perfumes such as alcohols, phenols, aldehydes, ketones, terpenes, and esters

When an volatile air treatment chemical is a disinfectant, preferred disinfectants include, but are not limited to, glycols, trimethylene and dipropylene. Organic acids compatible with the use of the substrate 22 and environment may also be used.

While the preferred embodiment of the present invention has been described above, it should be appreciated that the invention could be used in a variety of other embodiments. For example, the vent holes need not be placed on the transverse sides of the housing. Thus, the principles of the present invention can be applied in a wide variety of other ways apart from those specifically noted herein. Still other modifications may be made without departing from the spirit and scope of the invention. Thus, the claims (rather than just the preferred embodiment) should be reviewed in order to understand the full scope of the invention.

### Industrial Applicability

The present invention provides air treatment devices having efficient heater assemblies, which are easy to manufacture.

## Claims

1. An air treatment chemical dispensing device (10) that can, upon mounting a substrate (22) bearing a volatile air treatment chemical thereto, dispense the air treatment chemical from the substrate (22), the device (10) comprising:
a housing (12);
an electrical plug structure (14) mounted in the housing (12) with a portion extending rearwardly outward there from;
a heater (56) linked to the electrical plug structure (14) in the housing (12); and
a cover unit (18) covering the heater (56) and mounted to the housing (12);
wherein the cover unit (18) mounts the substrate (22) such that the substrate (22) has a first wall (65) of the substrate facing the heater (56) while a second wall of the substrate, on a side of the substrate opposite to the first wall (65), is essentially covered by the cover unit (18).

2. The device of claim 1, wherein the housing (12), electrical plug structure (14), heater (56) and cover unit (18) can be assembled by relative axial movement there between.

3. The device of claim 2, wherein a snap fit connection facilitates assembly of at least two of the housing (12), electrical plug structure (14), heater (56) and cover unit (18).

4. The device of claims 1, wherein the heater (56) is in a form of a table having a facing wall (61) and a side wall structure (63), wherein the heater (56) is capable of radiating heat from the facing wall (61) and the side wall structure (63) when the device (10) is assembled and powered by electricity.

5. The device of claim 1, wherein a portion of the heater (56) can be telescoped into the electrical plug structure (14).

6. A method for assembling the device of claim 1, comprising:
placing the electrical plug structure (14) in the housing (12) by relative axial movement, with a portion extending rearwardly outward there from;
placing the heater (56) in the housing by relative axial movement until it telescopes with the electrical plug structure (14); and
mounting the cover unit (18) to the housing (12) by relative axial movement while the cover unit (18) has the substrate positioned thereon.

7. An air treatment chemical dispensing device (10) that can, upon mounting a substrate (22) bearing a volatile air treatment chemical thereto, dispense the air treatment chemical from the substrate (22), the device (10) comprising:
a housing (12);
a heater (56) positioned in the housing (12), wherein the heater (56) is in a form of a table having a facing wall (61), wherein the heater is capable of radiating heat from at least the facing wall (61);
means for mounting the substrate (22) adjacent the facing wall (61) so that when heat is radiated from the facing wall it will radiate at least in part towards the substrate (22) to cause the substrate (22) to release the air treatment chemical at least partially towards the facing wall (61); and
an air pathway structure (30A, 30B) in and through the housing permitting air to pass into the housing (12) between the facing wall (61) and the substrate (22), if such a substrate (22) is mounted in the housing (12) by the means for mounting, and then out of the housing.

8. The device of claim 7, wherein the heater table (56) also has a side wall structure (63) capable of radiating heat there from into a portion of the air pathway structure.

9. The device of claim 7, wherein the air pathway structure (30A, 30B) comprises an inlet vent (30A) and an outlet vent (30B) located on opposed lateral sides of the device such that the air pathway structure extends transversely across the facing wall (61).

10. The device of claim 7, further comprising the substrate (22) mounted by the means for mounting in the housing (12).

11. The device of claim 10, wherein the substrate has a projecting nose (25) positioned adjacent the facing wall (61).

12. The device of claim 11, wherein the substrate has a projecting nose (25) positioned in contact with the facing wall (61).

13. The device of claim 10, wherein when the heater (56) is activated, the substrate (22) is heated thereby to release the air treatment chemical into air passing through the air pathway structure (30A, 30B).

14. The device of claim 7, wherein the volatile air treatment chemical is selected from the group consisting of insect control agents, fragrances, and deodorizers.

15. The device of claim 7, wherein the volatile air treatment chemical is an insect control agent, for example transfluthrin or metofluthrin.
